# EUROPEAN PATENT SPECIFICATION

(11) **EP 0 576 547 B1**
(45) Date of publication and mention of the grant of the patent: **10.06.1998**
(21) Application number: 92908122.2
(22) Date of filing: 12.03.1992
(51) Int. Cl.: C07C 51/47

(54) **PROCESSES FOR RECOVERING CITRIC ACID**
VERFAHREN ZUR WIEDERGEWINNUNG VON CITRONENSÄURE
PROCEDES DE RECUPERATION D'ACIDE CITRIQUE

(30) Priority: 14.03.1991 US 669490
(43) Date of publication of application: 05.01.1994
(73) Proprietor: REILLY INDUSTRIES, INC., Indianapolis, Indiana 46204 (US)
(72) Inventor: McQUIGG, Donald, Plainfield, IN 46168 (US); MARSTON, Charles, K., Midland, MI 48640 (US); FITZPATRICK, Gina, Indianapolis, IN 46254 (US); CROWE, Ernest, Beech Grove, IN 46107 (US); VORHIES, Susan, Indianapolis, IN 46214 (US)
(74) Representative: Bannerman, David Gardner
(86) International application number: US9201986
(87) International publication number: WO9216490

(56) References cited:
- EP-A- 0 324 210
- EP-A- 0 346 196
- GB-A- 868 926
- US-A- 4 851 573

## Description

This invention relates generally to processes for recovering citric acid, and more particularly to such processes involving adsorption and desorption of citric acid to and from a solid-phase polymer.

By way of further background, citric acid is a biologically occurring material which finds primary use in the food industry. It has gained worldwide acceptance as a food ingredient, and, has a pleasant acid taste and high water solubility which have motivated its most extensive food application in beverages, jams, jellies, and sweets. Additional uses of citric acid are also prevalent. For instance, it is used in the pharmaceutical and cosmetics industries, in the plastics industry as a raw material for the manufacture of citric acid ester plasticizers, as well as more recently finding substantial use in the preparation of detergents and other cleaning agents.

Since the early, work of C. Wehmer beginning in the 1890's, there has been substantial interest and investment in fermentation processes for producing citric acid. As such, nearly all of the 700 million pounds or more of citric acid produced worldwide yearly are from fermentation processes, for instance by the fermentation of a carbon source such as molasses with the microorganism, Aspergillus Niger. Typically, broths from such fermentations will contain about 10 weight% or more citric acid, as well as about 1000 ppm or more salts, about 1 weight% carbohydrates, and 2 weight% proteins, amino acids and other materials.

As will be appreciated, the recovery of the citric acid product from such mediums has itself been the subject of substantial attention in the academia and industry. In general, three techniques have been used to date, those being precipitation, solvent extraction and solid-phase polymer adsorption and subsequent desorption. As to the first technique, precipitation, it can be fairly stated that it has been the more preferred technique used on a commercial scale, even though significant endeavors as to the other two techniques have been made. In precipitation, calcium hydroxide (lime) is usually added to the fermented medium to form the slightly soluble tricalcium citrate tetrahydrate. Properly performed, this precipitation leaves most impurities in the solution. Impurities may further be removed by washing the filtered precipitate. To further purify the product, the moist precipitate is reacted with sulfuric acid to yield calcium sulfate (gypsum) and a solution of free acid. The free acid solution is then treated with activated carbon and ion exchange resins before evaporation to the crystalline citric acid product. As is recognized, the efficacy of this precipitation method is highly dependent on properly and carefully performing the various steps involved. It is thus a sensitive process requiring high refinement, especially on a commercial scale.

A second technique which has been used to recover citric acid is solvent extraction. In this technique, citric acid is extracted from the fermentation broth with solvent hydrocarbons, for example, octane, benzene, kerosene, ethers, esters, ketones or amines. Citric acid is then reextracted from the solvent phase into water with either the addition of heat or the formation of a citric acid salt. However, this solvent extraction technique is also expensive and complex. Further, solvent extraction generates a very substantial amount of waste for disposal, which from both cost and environmental standpoints is unattractive.

A third technique which has been suggested but to applicants' knowledge not applied on a commercial scale involves the use of solid adsorbents to remove citric acid from the medium. The adsorbed citric acid is then recovered from the polymer utilizing a desorbing agent. For example, U. S. Patent No. 4,323,702 to Kawabata et al. describes a process for recovering carboxylic acids with a material of which the main component is a polymeric compound having a pyridine skeletal structure and a cross-linked structure. As the patent directs, the captured carboxylic acids are then desorbed using an aliphatic alcohol, an aliphatic ketone or a carboxylic ester as the desorbing agent.

U. S. Patent No. 4,720,579 to Kulprathipanja describes a process in which citric acid is separated from a fermentation broth using an adsorbent of a neutral, noniogenic, macroreticular, water-insoluble cross-linked styrene-poly(vinyl)benzene. In this patent, a number of pulse tests were run using the described adsorbents. In these tests, a helical column was filled with the adsorbent, and a liquid described as the desorbent passed through the column. At a convenient time, a pulse of feed containing known concentrations of citric acid and other components was injected into the column. The manner in which the injected materials came off the column was then studied. For example, several tests were run in which water was passed through the column, although the patent cautioned that using water, increased desorption temperatures so as to cause premature deactivation of the adsorbent were required. Thus, the patent described a solution to this problem which included adding acetone in about 1 to 15% to the water as desorbent.

In U. S. Patent No. 4,851,573 to Kulprathipanja et al., another process is described in which an adsorbent of a cross-linked acrylic or styrene resin matrix having attached tertiary amine functional groups or pyridine functional groups is used as an adsorbent. Exemplified in the patent are again pulse tests similar to those described in the above-identified Kulprathipanja '579 patent, this time at temperatures of 60° to 75°C. The adsorbents exemplified included an acrylic resin having attached modified tertiary amines groups functionalized with sulfate ions, and polystyrene resins having attached pyridine groups functionalized with sulfate ions. The patent exemplified addition of sulfuric acid to citric acid solutions prior to the adsorption step, which helps drive functionalization to completion. The Kulprathipanja '573 patent further identifies sulfuric acid and other inorganic acids and water as desorbents, but directs a strong preference for the dilute sulfuric acid because others will be "found to be less effective" and specifically in some cases states that water "is not strong enough to recover the absorbed citric acid quickly enough to make the process commercially attractive."

In still another patent, U. S. Patent No. 4,851,574, Kulprathipanja describes separating citric acid from a fermentation broth using an adsorbent of a cross-linked acrylic or styrene resin matrix having attached aliphatic quaternary amine functional groups. Again, pulse tests are exemplified, in which sulfuric acid is demonstrated as a desorbent. These pulse tests were conducted at temperatures ranging from 50° to 60°C.

In light of the above and other literature, there remains a need for a highly commerically attractive process for recovering citric acid from a fermentation broth or other medium, by which the citric acid is recovered in a form that is readily purifiable and free from unnecessary organic or inorganic impurities which need be removed. The above-described work by Kawabata utilized organic solvents as desorbing agents which need be removed, thus adding expense and complication to the purification process. Moreover, where alcohol is used as desorbent, upon concentration of the desorbed medium or evaporation of solvent, significant esterification occurs thus leading to additional undesirable impurities in the product. The above-described work by Kulprathipanja et al. harbors instability, due to the resins used, and clearly directs that preferred desorbents include either inorganic acids or organic solvent materials which are processed through the column along with the citric acid product and thereby substantially contaminate the same and necessitate significant additional purification measures. Advantageous processes are therefore needed utilizing resins which minimize possible impurities in the product, as well as avoid the need to purify substantial amounts of inorganic acid desorbents such as sulfuric acid or organic desorbents such as aliphatic alcohols, ketones and carboxylic esters from the product. The applicants' invention addresses these needs and provides for the first time commercially attractive processes employing highly stable adsorbents as well as desorbent steps which minimize the opportunity for unnecessary impurities in the desorbed product and greatly simplify workup procedures.

### SUMMARY OF THE INVENTION

Accordingly, the present invention provides in one preferred embodiment a process for treating a medium to recover citric acid therein contained. This process includes contacting the medium with a solid-phase free base polymer having tertiary amine functions to adsorb the citric acid. The citric acid is then desorbed by displacement with a stronger acid, e.g. H₂SO₄ or HCl, and a citric acid-containing fraction is recovered substantially free from contamination by the stronger acid.

Another preferred embodiment of the invention provides a process for treating a medium to recover citric acid therein contained. The process includes the step of contacting the medium with a solid-phase free base polymer having pyridine functions at a temperature below about 40°C to adsorb citric acid on the polymer. Adsorbed citric acid is then desorbed with hot H₂O at a temperature of at least about 75°C.

The applicants have now surprisingly discovered that these inventive processes provide preparations of high commercial and technical attraction. The adsorbent pyridine and other free base resins exhibit good capacity as well as regenerability after performance of the process. In addition, the adsorption/desorption processes provide desorbate product mediums rich in citric acid without the presence of substantial organic solvents or inorganic acids in the desorbate which complicate further processing. Further, these processes have proven unexpectedly effective in reducing ash and readily carbonizable impurity content in the desorbed product.

These and additional features, objects and advantages of the invention will be apparent from reading the following description and appended claims.

### DESCRIPTION OF THE PREFERRED EMBODIMENTS

For the purposes of promoting an understanding of the principles of the invention, reference will now be made to certain preferred embodiments and specific language will be used to describe the same. It will nevertheless be understood that no limitation of the scope of the invention is thereby intended, such alterations, and further modifications or applications of the principles of the invention as described herein being contemplated as would normally occur to one skilled in the art to which the invention relates.

As indicated above, one preferred embodiment of the invention relates to a process for recovering citric acid from a medium in which it is contained. By this inventive process, the citric acid is recovered both effectively and in a form which is readily processible to obtain a purified citric acid product.

As to the medium itself, it will typically be a fermentation broth containing water, citric acid, salts, amino acids and other various components in minor amounts. Usually, the medium will contain citric acid at a level of about 10 wt% or more, and the salt concentration will typically be about 1000 ppm or more. Ordinarily, amino acids and other various materials account for about 2% by weight or less of the medium. As stated previously, the broth may be from a fermentation of a carbon source (e.g. carbohydrates such as corn sugar or molasses) with a suitable microorganism such as Aspergillus Niger. Other citric acid-containing mediums will also be suitable, and removal of citric acid therefrom is also contemplated as being within the spirit and scope of the present invention.

As to the method of contacting the medium and adsorbent, this can be done in any suitable manner as those practiced in the area will appreciate. For instance, either fixed, moving or fluidized bed systems can be used to provide batch, semi-continuous or continuous processes. A fixed bed is preferably alternately contacted with the medium and the water desorbent, each of which can be passed for example upflow or downflow through a resin bed. In a preferred mode, two or more fixed beds are provided in a system appropriately constructed and valved to reversibly contact one bed with the medium and another with desorbent and/or materials for rinsing or regenerating the resin. In this manner, continuous recovery processes can be conducted. Other contacting systems, for example countercurrent moving bed or simulated moving bed systems, can also be used within the skill of the ordinary artisan.

The adsorption step is preferably conducted at a temperature below about 40°C. At these lower temperatures, the medium containing citric acid is contacted with the free base polymer having tertiary amine functions which is highly effective for adsorbing the acid. The adsorption step is more desirably conducted at temperatures of about 25°C or below, for instance at near ambient temperatures or below, to maximize adsorption of citric acid by the resin. For lower temperatures, cooling can be provided as necessary to achieve the desired temperature. Of course, the temperature of the contacting in any event will be sufficiently high to prevent freezing of the medium. In more preferred processes, the medium is passed either upflow or downflow through a resin bed at a rate of about 2 to about 12 bed volumes per hour, and more preferably about 4 to 6 bed volumes/hour. The most desirable throughput will vary with the particular resin and other conditions used, as well as process economics involved, as will be determinable by those practiced in the field.

The medium containing citric acid is preferably passed through the resin bed for a time sufficient to achieve substantial or complete saturation of the bed. That is, until the bed has substantially or essentially ceased adsorbing citric acid from the medium. For instance, this resin saturation can be monitored by measuring the pH of the influent and effluent to and from a column containing the resin, with the resin being considered to be saturated when the influent and effluent exhibit the same or substantially, the same pH. In this mode of operation, there will of course be some citric acid which passes through and is not adsorbed to the resin, and the medium in which it is contained can be recycled into the column feed if desired.

The preferred free base polymer used in this embodiment of the invention exhibits superior capacities for adsorbing the citric acid. The polymer is solid-phase, that is, it remains a solid in each step of the recovery process including for instance the adsorption, rinse and desorption steps. In this regard, polymers having either N-aliphatic or N-heterocyclic tertiary amine functions can be used. For example, AMBERLYST® A-21 resin from Rohm and Haas, Philadelphia, Pennsylvania can be used in the invention. This A-21 resin contains aliphatic tertiary amine functions. For additional information about this and other similar resins, reference can be made to the literature including that available from the manufacturer. See, e.g., AMBERLYST A-21® technical bulletin fluid process chemicals," Rohm and Haas, April 1977. More preferred have been polyvinylpyridine polymers such as poly 2- and poly 4-vinylpyridine free base gel or macroreticular resins exhibiting a bead form. These resins are preferably at least about 2% cross-linked, and more preferably at least about 8% cross-linked with a suitable cross-linking agent, desirably divinylbenzene. More preferred resins to date have been 2 to 25% crosslinked bead-form poly 2- and poly 4-vinylpyridine polymers. For example, preferred polymers in work to date have been poly 2-and poly 4-vinylpyridine resins available from Reilly Industries, Inc., Indianapolis, Indiana, in the REILLEX™ polymer series. These REILLEX™ polymers are 2% or 25% crosslinked, and exhibit good thermal stability and adsorptive and desorptive capacities and other preferred features as described herein. For example, preferred resins of this type have exhibited desorptive capacities of at least about 200mg citric acid per gram of polymer. Additional preferred resins are available from this same source under the REILLEX™ HP polymer series. These REILLEX™ HP polymers exhibit advantageous capacity as demonstrated in Example 1 below. Further, REILLEX™ HP polymers have proven to be highly regenerable for example by processes similar to Example 6 below. For more information about these REILLEX™ polymers, reference can be made to the literature, including that available from Reilly Industries, Inc. in the form of REILLEX™ reports 1, 2 and 3, which are hereby incorporated by reference in all aspects relevant and material to the invention.

The preferred resin beads can be of any suitable mesh size, for instance preferably about 20 to 60 mesh. Further, the resins can include a minor amount of functionalization of their pyridine groups, which minor amount can include for example functionalization to pyridine N-oxide or quaternary salt species. This functionalization in the applicants' work has been incorporated to modify the relative basicity of the non-functionalized pyridine groups and thereby modify their adsorptive and desorptive properties.

As to the desorption step, as stated, it is performed in one embodiment using an acid stronger than citric acid, desirably H₂SO₄ or HCl. The stronger acid displaces the citric acid on the polymer adsorbent and the eluting citric acid is collected in a fraction free from any substantial contamination by the stronger acid (e.g. this fraction preferably contains about 5% by weight or less of the stronger acid, more preferably 1% or less). This can be achieved, for instance, by monitoring the pH of the influent and effluent from the polymer bed, and discontinuing the desorption when the effluent pH indicates significant presence of the stronger acid. As an example, when 5% H₂SO₄ is used as desorbent, the desorption can be stopped when the effluent pH drops below about 1.

In another preferred embodiment of the invention, where the free base polymer contains pyridine functions, the desorption is conducted with water at a temperature above about 75°C, with H₂O. In this regard, the 75°C temperature can be attained in any manner suitable. For example, the H₂O desorbent can simply be preheated to the necessary temperature prior to contacting with the resin. Additionally, as demonstrated in Examples 1-3 below, the water can be contacted with the resin in a suitable column or vessel having a jacket, and a hot fluid can be circulated through the jacket until the column internal temperature reaches the desired temperature. More preferred temperatures have been at least about 85°C, and temperatures even higher can be used, for instance temperatures of about 85-150°C and above can be used with steam and/or under pressure. For instance, Example 8 sets forth the results of a steam desorption of citric acid. As reported in the Example, after only 3 bed volumes of water (in the form of steam) had been passed through a citric-acid loaded resin, 33% of the citric acid had been removed.

In the applicants' preferred processes it is also desirable to rinse the polymer after it is saturated with citric acid and prior to the desorption step. In this regard, the rinse is preferably conducted with water, and at a temperature sufficiently low that no substantial loss of the citric acid from the resin is incurred during the rinse step. Preferred temperatures have been about 15°C or below, more preferably about 5°C or below. It is also desirable that the rinse water be adjusted to exhibit a pH of about 4.5 or less to help prevent undue loss of citric acid during the rinse step. In another feature of the invention, this pH adjustment is accomplished by adding CO₂ to the rinse water, for instance by adding dry ice to the water at low temperatures at which sufficient CO₂ is dissolved to achieve the desired pH. The rinse step is advantageously conducted with about 1 or more bed volumes of water, which are passed either upflow or downflow through the bed at a rate of about 15-20 bed volumes per hour. In any event, preferred rinse steps are conducted so as to remove at least substantially all of the salts or other non-adsorbed impurities which may be caught up in the resin bed.

In preferred processes the desorbent (hot water or stronger acid solution) is used in a volume so as to obtain a final desorbed medium containing at least about 2% by weight citric acid, and more preferably at least about 5% by weight citric acid. For example, in preferred hot water desorptions at about 85° to 100°C and using preferred resins, approximately 1.5 bed volumes of desorbent water and durations of about 5 to about 20 minutes have provided final desorbed mediums containing about 5% by weight or more citric acid. Similarly, using 10% sulfuric acid desorbent, about 3 bed volumes passed at a flow rate of about 4 to 6 bed volumes/hour have provided desorbed mediums containing about 5% or more citric acid. Of course, other factors will contribute to this finally-obtained concentration, for instance the particular resin used, its extent of saturation with citric acid, etc. Having the benefit of the disclosure herein, those skilled in the art will be readily able to manipulate these and other similar parameters to obtain the preferred citric acid levels in the final desorbed medium.

The preferred processes of the invention have also provided desorbed citric acid mediums of unexpectedly reduced levels of ash and readily carbonizable impurities (RCS). For example, preferred process have reduced ash and RCS content by at least about 65% each, more preferably at least about 80%. and in highly desirable processes about 90% or more. These qualities are highly significant from both technical and commercial standpoints, and further demonstrate the surprising nature and advantage of the applicants' discoveries.

While the invention has been described in detail in the foregoing paragraphs, the same is to be considered as illustrative and not restrictive in character, it being understood that only the preferred embodiments have been described and that all changes and modifications that come within the spirit of the invention are desired to be protected. The following specific Examples are given in further explanation and description of these embodiments, but are intended to be exemplary and not limiting thereof.

### EXAMPLE 1

### Desorption Capacity for REILLEX™425 and HP Resins

The citric acid desorption capacity was determined for various polyvinylpyridine polymers by first saturating the polymers with citric acid, desorbing the adsorbed citric acid with hot water, and measuring the amount of citric acid removed. Accordingly, 10 wt% citric acid solutions were prepared and their concentrations accurately determined by titrating with 0.1N NaOH to a phenolpthalein endpoint.

The resin to be tested was sieved to give a 20 to 60 mesh fraction. 30 to 50 cc portions of the resin were accurately weighed out and slurried with water in a jacketed 1" diameter ion-exchange column with volume markings. After the resin swelled and settled, the water level was lowered to the top of the resin bed and the resin's volume determined. Another sample of the resin from the same mesh fraction was weighed, dried to a constant weight at 70°C vacuum under reduced pressure, and its loss-on-drying (L.O.D.) determined. The L.O.D. was used to calculate the dry weight of resin packed in the column.

The prepared citric acid solution was passed downflow through the resin bed at a moderate rate (5 to 10 bed volumes an hour) until the resin was saturated as determined by the effluent concentration equaling that of the influent. The liquid level was drained to the top of the resin bed, and the resin was rinsed downflow with one bed volume of cold (≤ 5°C) CO₂ saturated water with a pH less than 4.5. This rinse water had been prepared by adding dry ice to water. Compressed air was blown through the bed to dry the resin. The entire column effluent and rinses were combined, purged with nitrogen to remove CO₂, and an aliquot titrated with 0.1 N sodium hydroxide to determine the citric acid concentration. The amount of citric acid in the combined effluent and rinses was determined from this figure, and subtracted from the total amount of citric acid introduced into the column. The resulting figure represented the amount of citric acid adsorbed by the resin.

For the desorption step, 1.5 bed volumes of water were added to the column, and the resin bed agitated with compressed air. Boiling water (100°C) was circulated through the jacket of the column for about 20 minutes until the internal temperature was at least 85°C. The column was drained and again blown dry with compressed air. After cooling to room temperature, the citric acid recovered from the resin was determined by titration. From this figure and the dry weight of the polymer on the column, the amount of citric acid desorbed per gram of resin was determined.

The results of this testing are shown in Table 1. As demonstrated, the hot water desorption process is highly effective using the polyvinylpyridine weak base polymers. For instance, both REILLEX™ 425 polymer ("R425") and REILLEX™ HP ("RHP") polymer samples exhibited good citric acid desorption capacity, with the former showing greater capacity in this work.

**TABLE 1**

| Resin | Run | % Acid(Infl.) | Acid Desorbed | Desorption Capacity |
|---|---|---|---|---|
| RHP^{a} | 1 | 10.2 | 4.17 g | 240 mg/g resin |
| | 2 | 10.6 | 3.60 g | 207 mg/g resin |
| | 3 | 10.1 | 4.06 g | 234 mg/g resin |
| | | | | |
| R425^{b} | 1 | 9.7 | 6.28 g | 302 mg/g resin |
| | 2 | 9.7 | 5.97 g | 287 mg/g resin |
| | 3 | 9.7 | 6.36 g | 306 mg/g resin |
| | | | | |
| R425^{c} | 1 | 10.4 | 3.31 g | 338 mg/g resin |
| | 2 | 10.4 | 3.27 g | 334 mg/g resin |
| | 3 | 10.4 | 3.23 g | 330 mg/g resin |

| | | | | |
|---|---|---|---|---|
| ^{a} Polymer dry wt. = 17.4 g. Water-wet polymer vol. = 62 cc. | | | | |
| ^{b} Polymer dry wt. = 20.8 g. Water-wet polymer vol. = 65 cc. | | | | |
| ^{c} Polymer dry wt. = 9.8 g. Water-wet polymer vol. = 34 cc. | | | | |

### EXAMPLE 2

### Desorption Testing For Various Resins

The procedure of Example 1 was repeated, except using the various gel and macroreticular ("macro") resins listed below. In each case the resins demonstrated the ability to effectively adsorb citric acid, and desorption capacities above 100 mg citric acid/g polymer were observed in all cases, and in most cases above 200 mg citric acid/g polymer. Poly(4-vinylpyridine) crosslinked with 8% divinylbenzene (resin No. 2) demonstrated the greatest desorption capacity at well over 300 mg citric acid/g resin. The partially quaternized and N-oxide for resins (Nos. 1 and 6, respectively) demonstrated improved reversibility over their non-modified counterparts (reversibility = desorbed grams citric acid/total grams citric acid adsorbed x 100) while still maintaining respectable desorption capacities.

**TABLE 2**

| No. | Vinylpyridine | Crosslinker/% | Type | Modification |
|---|---|---|---|---|
| 1 | poly-4-vinylpyridine | DVB/25%^{a} | macro | 22% quat.^{b} |
| | | | | |
| 2 | Poly-4-vinylpyridine | DVB/8% | gel | |
| | | | | |
| 3 | Poly-2-vinylpyridine | DVB/12% | macro | |
| | | | | |
| 4 | Poly-2-methyl- | | | |
| | 6-vinylpyridine | DVB/8% | gel | |
| | | | | |
| 5 | Poly-2-methyl- | | | |
| | 6-vinylpyridine | DVB/25% | gel | |
| | | | | |
| 6 | Poly-4-vinylpyridine | DVB/25% | macro | 25% N-oxide^{c} |
| | | | | |
| 7 | Poly-2-vinylpyridine | DVB/14% | gel | |
| | | | | |
| 8 | Hydrophylic Poly-4-vinylpyridine | DGD/14%^{d} | macro | |

| | | | | |
|---|---|---|---|---|
| ^{a} DVB = divinylbenzene | | | | |
| ^{b} 22 equivalent% methyl iodide | | | | |
| ^{c} 25 equivalent% N-oxide | | | | |
| ^{d} DGD = diethylene glycol dimethacrylate | | | | |

### EXAMPLE 3

### Desorptions Achieving High Citric Acid Concentration

In this experimentation, it was demonstrated that the desorbate could effectively be used in subsequent desorption steps to obtain mediums having advantageously high concentrations of citric acid. In these experiments, REILLEX™425 polymer was used in processes as described in Example 1, and the collected desorbed fluids were put back in the column after another saturation and rinse cycle, instead of water. Hot water was then again cycled through the jacket as in Example 1 until the internal column temperature reached at least 85°C. Using this technique, a concentration up to about 10% citric acid is achieved in two cycles. Additional cycles can be performed to further increase citric acid concentration, but in applicants work thus far, due to decreasing usable capacity of the resin with each cycle, the best efficiency has been achieved after two cycles.

### EXAMPLE 4

### Rate of Hot Water Desorption

Another set of experiments was conducted to determine the rate at which citric acid is desorbed from the resin using hot water. Accordingly, processes analogous to those described in Example 1 were performed up to the rinse step. After the rinse step, the resin was removed from the column and transferred to a stirred beaker containing 1.5 bed volumes of boiling water. 3 mL samples of the desorbate were taken at time intervals of 3, 5, 7, 10, 16, 22, and 30 minutes. The samples were weighed and titrated with 0.1N NaOH. The amount of citric acid in the total solution was calculated from the titration results. The fraction of citric acid desorbed at each interval was calculated, regarding the total amount of citric acid in solution at the final sample time as 100%. The results, presented in Table 3 below, demonstrate that the majority of the citric acid desorbed in the total 30 minute treatment was desorbed in the first 5 minutes.

**TABLE 3**

| Sample Time | % Desorbed |
|---|---|
| 3 minutes | 62 |
| 5 minutes | 80 |
| 7 minutes | 85 |
| 10 minutes | 89 |
| 16 minutes | 91 |
| 22 minutes | 95 |
| 30 minutes | 100 |

### EXAMPLE 5

### Effect of Salts on Desorption Capacity

Three experiments were done to determine the effect of nutrients (salts) on resin performance. REILLEX™425 polymer was used, and the procedure was the same as outlined above in Example 1 except salt was added to the citric acid solution before saturating the column. A first experiment was performed using 6000 ppm NaCl. Two additional experiments were performed using citric acid solutions with the following nutrient mixture:
38 ppm potassium chloride
50 ppm calcium(II) as chloride salt
70 ppm potassium phosphate, monobasic
0.10 ppm copper(II) as sulfate
0.10 ppm zinc(II) as sulfate
0.10 ppm iron(III) as ammonium citrate
500 ppm magnesium sulfate.

In each case, it was discovered that the resins retained at least about 70% of their desorption capacity under the conditions of this experimentation. Further, the effect of salt concentration did not appear to be significant from applicants' work.

Still another analysis was performed to determine how much ash, from the nutrients, is washed off the resin in the rinse step and thus excluded from the product stream. For these experiments, the ash in the influent in the above three salt experiments was compared to the ash in the desorbate. Each of the mixtures was reduced in ash conten± approximately 80-90%. This is highly advantageous as it decreases the load on a deashing unit and therefore decreases associated operating costs. Further, this can result in about a 10-fold longer use of the deashing unit before regeneration is necessary.

In another set of experiments, the procedure of Example 1 was again repeated except using corn sugar and molasses broth in respective runs instead of the 10% citric acid solution. Good desorption capacities were again demonstrated, with results being similar to those obtained with the salt-containing citric acid solution described above in this Example. Also, the readily carbonizable impurities ("RCS") contents of the desorbed products were consistently dramatically reduced, e.g. about 65% or more in molasses broth experiments and about 85-90% in the corn sugar broth experments.

### EXAMPLE 6

### Regeneration of Polymer

In this set of experiments, the ability to efficiently regenerate the resin after use in adsorption and desorption steps (as described in Example 1) was demonstrated. REILLEX™425 polymer was the polymer first employed in these experiments. Accordingly, 1 liter of 10% H₂SO₄ was passed through a column containing a used sample of this resin at 6 bed volumes per hour with intermittent agitation. The resin was then rinsed with water. 1 liter of 4% NaOH was then added, at a rate of 1 bed volume per hour with intermittent agitation. The resin was again rinsed with water, and the NaOH treatment repeated. The resin was left to soak in the 4% NaOH for 36 hrs, where after the resin was rinsed with excess water. In subsequent testing, the resin was shown to have recovered 99% of its initial capacity.

In another experiment, it was shown that the REILLEX™425 polymer used in previous adsorption and desorption steps (as in Example 1) could be regenerated to 100% of its initial capacity using an alternative simple procedure. Accordingly, the used REILLEX™425 polymer was treated in a column with 1 liter of 4% NaOH at 1 bed volume per hour, with intermittent agitation. Thereafter, the resin was rinsed with water until the effluent water was neutral. By this simple procedure, 100% of the initial capacity of the HP resin was regained.

### EXAMPLE 7

### Regeneration of Polymer

Reillex™425 polymer used in previous adsorption and desorption steps (e.g. Ex. 1) was backwashed with water for 5 minutes at 50% bed expansion. 145 Grams of a 4% NaOH solution per liter of resin was then passed through the column at a flow rate of 2-4 bed volumes per hour. The resin was then rinsed slowly by passing 1.5 bed volumes of water through the column at 2-4 bed volumes/hr. Subsequently, 4 bed volumes of water were passed through the resin at a rate of 16 bed volumes/hour. The resulting resin was found to have 100% of its initial capacity for citric acid.

### EXAMPLE 8

### Steam Desorption of Citric Acid

A steam desorption apparatus was constructed including a 1 1/2 inch outer diameter stainless steel pipe about 20 inches long. The upper end of the pipe was fitted with a valved steam inlet port and a pressure gauge. The lower end was fitted with a pressure regulation valve. A 14-inch stainless steel water cooled condenser was also attached below the pressure regulation valve. A fine stainless steel wire mesh was positioned in the lower end of the pipe to hold the polymer within the pipe. The apparatus was attached to a pressure regulated high pressure steam line.

One liter of a 10% aqueous solution of citric acid was prepared. 50.0 grams (dry basis) REILLEX™425 polymer were added to the solution and the solution stirred for about 3 hours at ambient temperature. The polymer was filtered off and washed with 250 ml of 5°C carbonated water. The filtrate was analyzed by titration, whereby it was determined that 60.5 grams of citric acid had been loaded onto the polymer (e.g. acid loading = grams acid in original solution less grams acid in filtrate). The loaded polymer was then placed into the steam desorption apparatus (filling about 2/3 the volume of the pipe), and the outer surface of the apparatus was then heated with live steam. After this, steam was passed through the apparatus at 10-15 psig and a flow rate of about 2 bed volumes per hour (as measured by rate of liquid collection). 100 ml fractions were collected, and each was titrated with 0.1N NaOH. Upon analysis, it was determined that 19.7 g of citric acid were recovered within the first 3 bed volumes of steam collected. This represents a 33% recovery of citric acid from the polymer in the first 3 bed volumes, and gave a citric acid fraction of greater than 5% by weight citric acid. The high efficacy of the steam desorption procedure was thus demonstrated.

### EXAMPLE 9

### Stronger Acid Desorption of Citric Acid

A column containing REILLEX™425 resin was prepared as in Example 1. A 10% citric acid solution was passed through the column until saturation, and the saturated resin rinsed with CO₂ water, also as in Example 1. A 10% solution of sulfuric acid was prepared and passed through the resin bed in the column while the pH of the column effluent was monitored. After the pH dropped to 1, flow was discontinued (at this point, a 150ml citric acid traction had been collected). HPLC analysis of the citric acid fraction indicated that it contained 11.3g of citric acid, and 1.6 q sulfuric acid. Thus > 99% of the adsorbed citric acid was removed from the polymer prior to any significant presence of the stronger acid in the effluent, and a 7.4 weight % citric acid desorbed product medium was obtained. Further, in similar experiments employing molasses and corn sugar broth instead of the prepared 10% citric acid solution, analysis of the products showed that both ash and RCS content were very substantially reduced. For example, ash content was consistently reduced by about 85-90% or more, and RCS content was consistently reduced by about 85%-95% or more. Similar results are obtained when AMBERLIST A-21® resin, commercially available from Rohm and Haas Co., Philadelphia, Pennsylvania, is used instead of REILLEX®425 resin. This inventive process thus provides highly efficient desorption of citric acid from the resin, and a citric acid-rich product medium substantially and unexpectedly free from contamination by the stronger acid or other impurities.

## Claims

1. A process for treating a medium to recover citric acid therein contained, comprising:
contacting the medium with a solid phase free base polymer having tertiary amine functions to absorb the citric acid;
passing a solution of an acid stronger than citric acid over said solid phase free base polymer to form an eluent containing citric acid desorbed by displacement with the stronger acid; and
collecting a citric acid-containing fraction from said eluent containing 5% or more by weight of citric acid, said fraction being collected prior to any significant presence of the stronger acid in the eluent and thus being substantially free from contamination by the stronger acid.

2. A process according to claim 1, wherein said free base polymer having tertiary amine functions is a poly 2- or poly 4-vinylpyridine polymer.

3. A process according to claim 2, wherein said poly 2-or poly 4-vinylpyridine polymer is a crosslinked bead-form gel or macroreticular resin.

4. A process according to claim 3, wherein said poly 2-or poly 4-vinylpyridine polymer is at least 2% crosslinked with divinylbenzene.

5. A process according to claim 4, wherein said free base polymer having tertiary amine functions is a poly 2-vinylpyridine polymer.

6. A process according to claim 4, wherein said free base polymer having tertiary amine functions is a poly 4-vinylpyridine polymer.

7. A process according to claim 4, wherein said adsorbing is at a temperature below 25°C.

8. A process according to claim 7, and also including the step of rinsing said free base polymer having tertiary amine functions with an aqueous rinse medium between said adsorption and desorption steps.

9. A process according to claim 8, wherein said rinsing is at a temperature below 15°C and said aqueous rinse medium exhibits a pH below 4.5.

10. A process according to claim 9, wherein said aqueous rinse medium contains CO₂.

11. A process for effectively recovering citric acid i11 a readily purifiable form from a medium in which it is contained, comprising contacting said medium with a solid-phase divinylbenzene-crosslinked free base polymer having pyridine or tertiary aliphatic amine functions at a temperature below 40°C to adsorb citric acid on the polymer, and desorbing the adsorbed citric acid with hot H₂O at a temperature above 75°C.

12. A process according to claim 11, wherein said free base polymer having tertiary amine functions is a poly 2- or poly 4-vinylpyridine polymer.

13. A process according to Claim 12, wherein said desorbing is at a temperature of at least 85°C.

14. A process according to claim 13, wherein said poly 2- or poly 4-vinylpyridine polymer is a crosslinked polymer.

15. A process according to claim 14, wherein said poly 2- or poly 4-vinylpyridine is a bead-form gel or macroreticular resin.

16. A process according to claim 14, wherein said poly 2- or poly 4-vinylpyridine polymer is crosslinked with divinylbenzene.

17. A process according to claim 16, wherein said poly 2- or poly 4-vinylpyridine polymer is at least 8% crosslinked with divinylbenzene.

18. A process according to claim 17, wherein said free base polymer having tertiary amine functions is a poly 2-vinylpyridine polymer.

19. A process according to claim 17, wherein said free base polymer having tertiary amine functions is a poly 4-vinylpyridine polymer.

20. A process according to claim 17, wherein said poly 2- or poly 4-vinylpyridine polymer is a gel resin.

21. A process according to claim 17, wherein said poly 2- or poly 4-vinylpyridine polymer is a macroreticular resin.

22. A process according to claim 17, wherein said poly 2- or poly 4-vinylpyridine polymer has a desorption capacity of at least 200 milligrams citric acid per gram polymer.

23. A process according to claim 17, wherein at least 25% of the citric acid adsorbed on the polymer is desorbed during said desorbing step.

24. A process according to claim 22, wherein at least about 25% of the citric acid adsorbed on the polymer is desorbed during said desorbing step.

25. A process according to claim 23, wherein said adsorbing is at a temperature below 25°C.

26. A process according to claim 25, wherein said desorbing step provides an aqueous medium containing at least 2% by weight citric acid.

27. A process according to claim 26, wherein said free base polymer having tertiary amine functions also contains pendant pyridine N-oxide groups.

28. A process according to claim 26, wherein said polyvinylpyridine polymer also contains pendant quaternized pyridine groups.

29. A process according to claim 26, and also including the step of rinsing said free base polymer having tertiary amine functions with an aqueous rinse medium between said adsorption and desorption steps.

30. A process according to claim 29, wherein said rinsing is at a temperature below 15°C and said aqueous rinse medium exhibits a pH below 4.5.

31. A process according to claim 30, wherein said aqueous rinse medium contains CO₂.

32. A process according to claim 26, wherein said desorption step is conducted using the desorbate medium of a previous desorption step to increase the amount of citric acid in said desorbate medium.

33. A process according to claim 26, wherein said free base polyvinylpyridine polymer is a poly 4-vinylpyridine crosslinked with about 25% divinylbenzene exhibiting a macroreticular bead form.

34. A process according to claim 26, wherein said free base polyvinylpyridine polymer is a poly 4-vinylpyridine crosslinked with about 8% divinylbenzene exhibiting a gel bead form.

## Patentansprüche

1. Verfahren zur Behandlung eines Mediums, um darin enthaltende Zitronensäure zu gewinnen, welches umfaßt:
Kontakt des Mediums mit einem Polymer mit freien Basen in fester Phase, das tertiäre Aminfunktionen aufweist, wodurch die Zitronensäure absorbiert wird,
Leiten einer Lösung einer Säure, die stärker als Zitronensäure ist, über das Polymer mit freien Basen in fester Phase, wodurch ein Eluent gebildet wird, das durch Verdrängung mit der stärkeren Säure desorbierte Zitronensäure enthält, und
Auffangen der Zitronensäure enthaltenden Fraktion aus dem Eluent, die 5 Gew.-% oder mehr Zitronensäure enthält, wobei die Fraktion aufgefangen wird, bevor die stärkere Säure signifikant im Eluent auftritt, und somit im wesentlichen nicht mit der stärkeren Säure verunreinigt ist.

2. Verfahren nach Anspruch 1, wobei das Polymer mit freien Basen, das tertiäre Amionfunktionen aufweist, ein Poly(2-vinylpyridin)- oder Poly(4-vinylpyridin)-Polymer ist.

3. Verfahren nach Anspruch 2, wobei das Poly(2-vinylpyridin)- oder Poly(4-vinylpyridin)-Polymer ein vernetztes, kugelförmiges Gel oder makroretikuläres Harz ist.

4. Verfahren nach Anspruch 3, wobei das Poly(2-vinylpyridin)- oder Poly(4-vinylpyridin)-Polymer zu mindestens 2% mit Divinylbenzol vernetzt ist.

5. Verfahren nach Anspruch 4, wobei das Polymer mit freien Basen, das tertiäre Aminfunktionen aufweist, ein Poly(2-vinylpyridin)-Polymer ist.

6. Verfahren nach Anspruch 4, wobei das Polymer mit freien Basen, das tertiäre Aminfunktionen aufweist, ein Poly(4-vinylpyridin)-Polymer ist.

7. Verfahren nach Anspruch 4, wobei das Adsorbieren bei einer Temperatur von weniger als 25°C erfolgt.

8. Verfahren nach Anspruch 7, das zwischen den Adsorptions- und Desorptionsschritten auch den Schritt des Spülens des Polymers mit freien Basen, das tertiäre Aminfunktionen aufweist, mit einem wäßrigen Spülmedium umfaßt.

9. Verfahren nach Anspruch 8, wobei das Spülen bei einer Temperatur unter 15°C erfolgt und das wäßrige Spülmedium einen pH-Wert von weniger als 4,5 hat.

10. Verfahren nach Anspruch 9, wobei das wäßrige Spülmedium CO₂ enthält.

11. Verfahren zur wirksamen Gewinnung von Zitronensäure in leicht zu reinigender Form aus einem Medium, in dem sie enthalten ist, das den Kontakt des Mediums mit einem mit Divinylbenzol vernetzten Polymer mit freien Basen in fester Phase, das Pyridin- oder tertiäre aliphatische Aminofunktionen aufweist, bei einer Temperatur unter 40°C, wodurch die Zitronensäure auf dem Polymer adsorbiert wird, und das Desorbieren der adsorbierten Zitronensäure mit heißem H₂O bei einer Temperatur von mehr als 75°C umfaßt.

12. Verfahren nach Anspruch 11, wobei das Polymer mit freien Basen, das tertiäre Amionfunktionen aufweist, ein Poly(2-vinylpyridin)- oder Poly(4-vinylpyridin)-Polymer ist.

13. Verfahren nach Anspruch 12, wobei das Desorbieren bei einer Temperatur von mindestens 85°C erfolgt.

14. Verfahren nach Anspruch 13, wobei das Poly(2-vinylpyridin)- oder Poly(4-vinylpyridin)-Polymer ein vernetztes Polymer ist.

15. Verfahren nach Anspruch 14, wobei das Poly(2-vinylpyridin) oder Poly(4-vinylpyridin) ein kugelförmiges Gel oder makroretikuläres Harz ist.

16. Verfahren nach Anspruch 14, wobei das Poly(2-vinylpyridin)- oder Poly(4-vinylpyridin)-Polymer mit Divinylbenzol vernetzt ist.

17. Verfahren nach Anspruch 16, wobei das Poly(2-vinylpyridin)- oder Poly(4-vinylpyridin)-Polymer zu mindestens 8% mit Divinylbenzol vernetzt ist.

18. Verfahren nach Anspruch 17, wobei das Polymer mit freien Basen, das tertiäre Aminfunktionen aufweist, ein Poly(2-vinylpyridin)-Polymer ist.

19. Verfahren nach Anspruch 17, wobei das Polymer mit freien Basen, das tertiäre Aminfunktionen aufweist, ein Poly(4-vinylpyridin)-Polymer ist.

20. Verfahren nach Anspruch 17, wobei das Poly(2-vinylpyridin)- oder Poly(4-vinylpyridin)-Polymer ein Gelharz ist.

21. Verfahren nach Anspruch 17, wobei das Poly(2-vinylpyridin)- oder Poly(4-vinylpyridin)-Polymer ein makroretikuläres Harz ist.

22. Verfahren nach Anspruch 17, wobei das Poly(2-vinylpyridin)- oder Poly(4-vinylpyridin)-Polymer eine Desorptionskapazität von mindestens 200 mg Zitronensäure pro Gramm Polymer aufweist.

23. Verfahren nach Anspruch 17, wobei beim Desorptionsschritt mindestens 25% der auf dem Polymer adsorbierten Zitronensäure desorbiert werden.

24. Verfahren nach Anspruch 22, wobei beim Desorptionsschritt mindestens etwa 25% der auf dem Polymer adsorbierten Zitronensäure desorbiert werden.

25. Verfahren nach Anspruch 23, wobei das Adsorbieren bei einer Temperatur unter 25°C erfolgt.

26. Verfahren nach Anspruch 25, wobei der Desorptionsschritt ein wäßriges Medium liefert, das mindestens 2 Gew.-% Zitronensäure enthält.

27. Verfahren nach Anspruch 26, wobei das Polymer mit freien Basen, das tertiäre Aminfunktionen aufweist, auch gebundene Pyridin-N-oxid-Gruppen enthält.

28. Verfahren nach Anspruch 26, wobei das Polyvinylpyridin-Polymer auch gebundene quaternisierte Pyridingruppen enthält.

29. Verfahren nach Anspruch 26, das zwischen den Adsorptions- und Desorptionsschritten auch den Schritt des Spülens des Polymers mit freien Basen, das tertiäre Aminfunktionen aufweist, mit einem wäßrigen Spülmedium umfaßt.

30. Verfahren nach Anspruch 29, wobei das Spülen bei einer Temperatur unter 15°C erfolgt und das wäßrige Spülmedium einen pH-Wert von weniger als 4,5 hat.

31. Verfahren nach Anspruch 30, wobei das wäßrige Spülmedium CO₂ enthält.

32. Verfahren nach Anspruch 26, wobei der Desorptionsschritt mit dem Desorbatmedium eines vorangegangenen Desorptionsschrittes erfolgt, damit die Zitronensäuremenge im Desorbatmedium erhöht wird.

33. Verfahren nach Anspruch 26, wobei das Polyvinylpyridin-Polymer mit freien Basen ein mit etwa 25% Divinylbenzol vernetztes Poly(4-vinylpyridin) ist, das eine makroretikuläre Kugelform hat.

34. Verfahren nach Anspruch 26, wobei das Polyvinylpyridin-Polymer mit freien Basen ein mit etwa 8% Divinylbenzol vernetztes Poly(4-vinylpyridin) ist, das als kugelförmiges Gel vorliegt.

## Revendications

1. Procédé pour le traitement d'un agent pour récupérer l'acide citrique contenu dans celui-ci, comprenant :
mise en contact de l'agent avec un polymère à base libre en phase solide possédant des fonctions d'amine tertiaire pour absorber l'acide citrique ;
faire passer une solution d'un acide plus fort que l'acide citrique sur le polymère à base libre en phase solide pour former un éluant contenant de l'acide citrique désorbé par déplacement avec l'acide plus fort ; et
recueillir une fraction contenant l'acide citrique à partir de l'éluant contenant 5 % en poids ou davantage d'acide citrique, la fraction étant recueillie avant la présence significative de l'acide plus fort dans l'éluant et étant ainsi sensiblement exempte de contamination par l'acide plus fort.

2. Procédé selon la revendication 1, dans lequel le polymère à base libre ayant des fonctions d'amine tertiaire est un poly 2- ou poly 4-vinylpyridine polymère.

3. Procédé selon la revendication 2, dans lequel le poly 2- ou poly 4-vinylpyridine polymère est un gel qui se présente sous la forme de perles réticulées ou une résine macroréticulaire.

4. Procédé selon la revendication 3, dans lequel le poly 2- ou poly 4-vinylpyridine polymère est au moins réticulé à 2 % avec du divinylbenzène.

5. Procédé selon la revendication 4, dans lequel le polymère à base libre possédant des fonctions d'amine tertiaire est un poly 2-vinylpyridine polymère.

6. Procédé selon la revendication 4, dans lequel le polymère à base libre possédant des fonctions d'amine tertiaire est un poly 4-vinylpyridine polymère.

7. Procédé selon la revendication 4, dans lequel l'adsorption se produit à une température au-dessous de 25°C.

8. Procédé selon la revendication 7, comprenant également l'étape consistant à rincer le polymère à base libre possédant des fonctions d'amine tertiaire avec un agent de rinçage aqueux entre les étapes d'adsorption et de désorption.

9. Procédé selon la revendication 8, dans lequel le rinçage s'effectue à une température inférieure à 15°C et l'agent de rinçage aqueux présente un pH au-dessous de 4,5.

10. Procédé selon la revendication 9, dans lequel l'agent de rinçage aqueux contient du CO₂.

11. Procédé pour récupérer efficacement l'acide citrique dans une forme facilement purifiable à partir d'un agent dans lequel il est contenu, comprenant la mise en contact de l'agent avec un polymère à base libre réticulé-divinylbenzène en phase solide possédant des fonctions d'amine aliphatique tertiaire ou pyridine à une température au-dessous de 40°C pour adsorber l'acide citrique sur le polymère, et désorber l'acide citrique adsorbé avec de l'H₂O brûlant à une température au-dessus de 75°C.

12. Procédé selon la revendication 11, dans lequel le polymère à base libre possédant des fonctions d'amine tertiaire est un poly 2- ou poly 4-vinylpyridine polymère.

13. Procédé selon la revendication 12, dans lequel la désorption se produit à une température d'au moins 85°C.

14. Procédé selon la revendication 13, dans lequel le poly 2- ou le poly 4-vinylpyridine polymère est un polymère réticulé.

15. Procédé selon la revendication 14, dans lequel la poly 2- ou poly 4-vinylpyridine est un gel en forme de perles ou une résine macroréticulaire.

16. Procédé selon la revendication 14, dans lequel le poly 2- ou poly 4-vinylpyridine polymère est réticulé avec du divinylbenzène.

17. Procédé selon la revendication 16, dans lequel le poly 2- ou le poly 4-vinylpyridine polymère est au moins réticulé à 8 % avec du divinylbenzène.

18. Procédé selon la revendication 17, dans lequel le polymère à base libre possédant des fonctions d'amine tertiaire est un poly 2-vinylpyridine polymère.

19. Procédé selon la revendication 17, dans lequel le polymère à base libre possédant des fonctions d'amine tertiaire est un poly 4-vinylpyridine polymère.

20. Procédé selon la revendication 17, dans lequel le poly 2- ou poly 4-vinylpyridine polymère est une résine en gel.

21. Procédé selon la revendication 17, dans lequel le poly 2- ou poly 4-vinylpyridine polymère est une résine macroréticulaire.

22. Procédé selon la revendication 17, dans lequel le poly 2- ou poly 4-vinylpyridine polymère présente une capacité de désorption d'au moins 200 mg d'acide citrique par gramme de polymère.

23. Procédé selon la revendication 17, dans lequel au moins 25 % de l'acide citrique adsorbé sur le polymère est désorbé pendant l'étape de désorption.

24. Procédé selon la revendication 22, dans lequel au moins environ 25 % de l'acide citrique adsorbé sur le polymère est désorbé pendant l'étape de désorption.

25. Procédé selon la revendication 23, dans lequel l'adsorption se produit à une température au-dessous de 25°C.

26. Procédé selon la revendication 25, dans lequel l'étape de désorption fournit un milieu aqueux contenant au moins 2 % en poids d'acide citrique.

27. Procédé selon la revendication 26, dans lequel le polymère à base libre possédant des fonctions d'amine tertiaire contient également des groupes N-oxyde pyridine en suspension.

28. Procédé selon la revendication 26, dans lequel le polyvinylpyridine polymère contient également des groupes pyridine quaternisés en suspension.

29. Procédé selon la revendication 26, et comprenant également l'étape consistant à rincer le polymère à base libre possédant des fonctions d'amine tertiaire avec un milieu de rinçage aqueux entre les étapes d'adsorption et de désorption.

30. Procédé selon la revendication 29, dans lequel le rinçage s'effectue à une température au-dessous de 15°C et l'agent de rinçage aqueux présente un pH au-dessous de 4,5.

31. Procédé selon la revendication 30, dans lequel l'agent de rinçage aqueux contient du CO₂.

32. Procédé selon la revendication 26, dans lequel l'étape de désorption est conduite en utilisant l'agent désorbé d'une étape de désorption préalable pour augmenter la quantité d'acide citrique dans l'agent de désorption.

33. Procédé selon la revendication 26, dans lequel le polyvinylpyridine polymère à base libre est une poly 4-vinylpyridine réticulée avec environ 25 % de divinylbenzène présentant une forme de perles macroréticulaires.

34. Procédé selon la revendication 26, dans lequel le polyvinylpyridine polymère à base libre est une poly 4-vinylpyridine réticulée avec environ 8 % de divinylbenzène présentant une forme de perles en gel.
